# EUROPEAN PATENT SPECIFICATION

(11) **EP 3 754 336 B1**
(45) Date of publication and mention of the grant of the patent: **17.01.2024**
(21) Application number: 19753825.9
(22) Date of filing: 08.02.2019
(51) Int. Cl.: G01N 33/68, A61K 33/22, C12Q 1/02

(54) **CANCER LESION TISSUE EVALUATION FOR OPTIMIZING EFFECT OF BORON NEUTRON CAPTURE THERAPY**
KREBSLÄSIONSGEWEBEAUSWERTUNG ZUR OPTIMIERUNG DER WIRKUNG VON BORNEUTRONENEINFANGTHERAPIE
ÉVALUATION DE TISSU DE LÉSION CANCÉREUSE POUR OPTIMISER L'EFFET D'UNE THÉRAPIE PAR CAPTURE DE NEUTRONS DE BORE

(30) Priority: 15.02.2018 JP 2018025216
(43) Date of publication of application: 23.12.2020
(73) Proprietor: 3-D Matrix, Ltd., Chiyoda-ku Tokyo 102-0083 (JP)
(72) Inventor: MATSUI, Hideki, Okayama-shi, Okayama 700-8530 (JP); FUJIMURA, Atsushi, Okayama-shi, Okayama 700-8530 (JP)
(74) Representative: Vossius & Partner Patentanwälte Rechtsanwälte mbB
(86) International application number: PCT/JP2019/005727
(87) International publication number: WO 2019/160129

(56) References cited:
- WO-A1-2017/026276
- WO-A1-2017/164334
- WO-A2-2005/051905
- WO-A2-2006/138275
- JP-A- 2013 087 098
- JP-A- 2016 020 316
- SASAI MASAO ET AL: "Novel Hyaluronan Formulation Enhances the Efficacy of Boron Neutron Capture Therapy for Murine Mesothelioma", ANTICANCER RESEARCH, vol. 36, no. 3, 14 March 2016 (2016-03-14) , pages 907-912, XP055809687, Retrieved from the Internet: URL:https://ar.iiarjournals.org/content/an ticanres/36/3/907.full.pdf>
- MAROUAN RAMI ET AL: "Sulfonamides incorporating boroxazolidone moieties are potent inhibitors of the transmembrane, tumor-associated carbonic anhydrase isoforms IX and XII", BIOORGANIC & MEDICINAL CHEMISTRY LETTERS, ELSEVIER, AMSTERDAM, NL, vol. 21, no. 10, 14 March 2011 (2011-03-14), pages 2975-2979, XP028208762, ISSN: 0960-894X, DOI: 10.1016/J.BMCL.2011.03.055 [retrieved on 2011-03-21]
- FENG B ET AL: "Delivery of sodium borocaptate to glioma cells using immunoliposome conjugated with anti-EGFR antibodies by ZZ-His", BIOMATERIALS, ELSEVIER, AMSTERDAM, NL, vol. 30, no. 9, 1 March 2009 (2009-03-01), pages 1746-1755, XP025928042, ISSN: 0142-9612, DOI: 10.1016/J.BIOMATERIALS.2008.12.010 [retrieved on 2009-01-01]
- DATABASE INSPEC [Online] THE INSTITUTION OF ELECTRICAL ENGINEERS, STEVENAGE, GB; November 2015 (2015-11), KETTENBACH K ET AL: "Synthesis and evaluation of boron folates for Boron-Neutron-Capture-Therapy (BNCT)", XP002804282, Database accession no. 15792021
- YEH CHUN-NAN ET AL: "Synthesis and characterization of boron fenbufen and its F-18 labeled homolog for boron neutron capture therapy of COX-2 overexpressed cholangiocarcinoma", EUROPEAN JOURNAL OF PHARMACEUTICAL SCIENCES, ELSEVIER AMSTERDAM, NL, vol. 107, 17 July 2017 (2017-07-17), pages 217-229, XP085151559, ISSN: 0928-0987, DOI: 10.1016/J.EJPS.2017.07.019
- SIHEM OUASTI ET AL: "The CD44/integrins interplay and the significance of receptor binding and re-presentation in the uptake of RGD-functionalized hyaluronic acid", BIOMATERIALS, ELSEVIER, AMSTERDAM, NL, vol. 33, no. 4, 6 October 2011 (2011-10-06), pages 1120-1134, XP028115489, ISSN: 0142-9612, DOI: 10.1016/J.BIOMATERIALS.2011.10.009 [retrieved on 2011-10-14]
- Gabel, D.; Neumann, M.; Bergmann, M: "BSH Accumulates Selectively in GFAP-positive Cells", Research and Development in Neutron Capture Therapy, Proceedings of the International Congress on Neutron Capture Therapy, 1 January 2002 (2002-01-01), pages 419-423, XP009522692, Italy
- DIO MEO, C. et al.: "Hyaluronan as Carrier of Carboranes for Tumor Targeting in Boron Neutron Capture Therapy", Biomacromolecules, vol. 8, no. 2, 2007, pages 552-559, XP055142431, DOI: 10.1021/bm0607426
- NAOR D ET AL: "CD44:STRUCTURE, FUNCTION, AND ASSOCIATION WITH THE MALIGNANT PROCESS", ADVANCES IN CANCER RESEARCH; [ADVANCES IN CANCER RESEARCH; ISSN 0065-230X; VOL], ACADEMIC PRESS, US, vol. 71, 1 January 1997 (1997-01-01), pages 241-319, XP000952636, ISSN: 0065-230X, DOI: 10.1016/S0065-230X(08)60101-3
- Skandalis Spyros S. ET AL: "Proteomic identification of CD44 interacting proteins", IUBMB Life, vol. 62, no. 11, 1 November 2010 (2010-11-01), pages 833-840, XP093007788, ISSN: 1521-6543, DOI: 10.1002/iub.392
- MUTSUKO OMATSU ET AL: "Immunohistochemical analysis of thymic carcinoma focusing on the possibility of molecular targeted and hormonal therapies", GENERAL THORACIC AND CARDIOVASCULAR SURGERY, SPRINGER JAPAN, JAPAN, vol. 60, no. 12, 3 October 2012 (2012-10-03), pages 803-810, XP035151001, ISSN: 1863-6713, DOI: 10.1007/S11748-012-0160-X

## Description

### Technical Field

The present invention relates to a method for predicting the effect of boron neutron capture therapy (BNCT) and use of a kit therefor.

### Background Art

BNCT is a method of cancer treatment by administering an agent containing boron (a boron agent) to a cancer patient, selectively incorporating a boron atom into the cancer cell, and irradiating neutrons to the affected area. In this method, a boron atom in the boron agent captures a neutron and splits into an alpha particle (a helium atomic nucleus) and a lithium atomic nucleus. Cancer cells are selectively destroyed by the energy of the alpha particle. Currently, p-boronophenylalanine (BPA) is mainly used in BNCT as a boron agent. However, there are many carcinomas that have not been found to yield sufficient results with BNCT using conventional BPA or that have not been considered to date a good indication for BNCT.

To predict the dosing effect of BPA, tissue aggregation of F-BPA has been evaluated by PET imaging, utilizing that F-BPA to which a radioisotope of fluorine is chemically bound is taken up in the same manner as BPA from amino acid transporters such as LAT1 (see, for example, Non Patent Literatures 1 to 5). However, because of the limited facilities available for PET imaging and the high price of the agents, it is not easy to predict the effects. In addition, no effective means have been found to evaluate the uptake into cells of boron agents other than BPA.

### Citation List

### Non Patent Literature

Non Patent Literature 1: EJNMMI Res. 2014 Dec 20; 4(1): 70. doi: 10.1186/s13550-014-0070-2. eCollection 2014 Dec.
Non Patent Literature 2: J Nucl Med. 2004 Feb;45(2):302-8.

### Non Patent Literature 3: J Nucl Med. 2005 2005

Nov;46(11) :1858-65.
Non Patent Literature 4: Ann Nucl Med. 2016 Dec;30(10):749-755. Epub 2016 Sep 1.
Non Patent Literature 5: Appl Radiat Isot. 2009 Jul;67(7-8 Suppl):S348-50. doi: 10.1016/j.apradiso.2009.03.061. Epub 2009 Mar 27.

### Summary of Invention

### Problem to be Solved

It has been required to perform effective BNCT for carcinomas that have not been found to yield sufficient results by BNCT using conventional BPA or that have not been considered to date a good indication for BNCT. For that, methods are needed to easily predict the effects of boron agents that differ from BPA. It is also required to select the optimal boron formulation for each carcinoma/case.

### Means to solve the Problem

In order to solve the above problem, the present inventors conducted intensive studies and have found that certain boron agents, a complex comprising a peptide containing a basic amino acid residue and mercaptoundecahydrodecaborate (BSH), and BSH to which a peptide containing a basic amino acid residue is covalently linked, are taken into a cancer cell through a pathway via CD44, and that BSH, a complex comprising BSH, or a BSH derivative targets a translation system in the cell, leading to the completion of the present invention.

That is, the present invention provides the following.
(1) A method for predicting efficiency of introduction into a cancer cell of a complex comprising a peptide containing a basic amino acid residue and BSH, or BSH to which a peptide containing a basic amino acid residue is covalently linked, the method comprising examining expression of CD44 in the cancer cell in a sample, wherein the peptide containing a basic amino acid residue contains an arginine residue and/or a lysine residue and is 2 to 20 amino acids in length.
(2) The method according to (1), wherein the expression of CD44 is examined by immunostaining.
(3) Use of a kit for predicting efficiency of introduction into a cancer cell of a complex comprising a peptide containing a basic amino acid residue and BSH, or BSH to which a peptide containing a basic amino acid residue is covalently linked, the kit comprising means for examining expression of CD44.
(4) A method for predicting a sensitivity of a cancer cell to a boron neutron capture therapy (BNCT) using a complex comprising a peptide containing a basic amino acid residue and BSH, or BSH to which a peptide containing a basic amino acid residue is covalently linked, the method comprising examining expression of CD44 in the cancer cell in a sample, wherein the peptide containing a basic amino acid residue contains an arginine residue and/or a lysine residue and is 2 to 20 amino acids in length.
(5) The method according to (4), wherein the expression of CD44 is examined by immunostaining.
(6) Use of a kit for determining a sensitivity of a cancer cell to BNCT using a complex comprising a peptide containing a basic amino acid residue and BSH, or BSH to which a peptide containing a basic amino acid residue is covalently linked, the kit comprising means for examining expression of an anti-CD44 antibody.
(7) A method for predicting a possibility that BNCT is effective using a complex comprising a peptide containing a basic amino acid residue and BSH, or BSH to which a peptide containing a basic amino acid residue is covalently linked, the method comprising: examining an expression of CD44 in a cancer cell in a sample; and predicting that the higher the expression of CD44, the higher the possibility that BNCT is effective using a complex comprising a peptide containing a basic amino acid residue and BSH, or BSH to which a peptide containing a basic amino acid residue is covalently linked.
(8) Use of a kit for predicting a possibility that BNCT is effective using a complex comprising a peptide containing a basic amino acid residue and BSH, or BSH to which a peptide containing a basic amino acid residue is covalently linked, the kit comprising means for examining expression of CD44.
(9) A method for selecting a boron formulation for BNCT, comprising the steps of:
   (a) examining expression of CD44 and expression of LAT1 in a cancer cell in a sample; and then
   (b) selecting a boron formulation containing a complex comprising a peptide containing a basic amino acid residue and BSH, or BSH to which a peptide containing a basic amino acid residue is covalently linked when the expression of CD44 is higher than the expression of LAT1, and selecting a boron formulation containing BPA when the expression of LAT1 is higher than the expression of CD44.
(10) Use of a kit for use in selecting a boron formulation for BNCT according to the method of (9), the kit comprising means for examining expression of CD44 and means for examining expression of LAT1.

### Effects of the Invention

According to the present invention, there are provided a method and a use of a kit for predicting an effect of BNCT when a complex comprising a peptide containing a basic amino acid residue and BSH, or BSH to which a peptide containing a basic amino acid residue is covalently linked are used as a boron agent. As a result, it is possible to expand BNCT indication cases and improve BNCT response rates, by the use of a complex comprising a peptide containing a basic amino acid residue and BSH, and BSH to which a peptide containing a basic amino acid residue is covalently linked for carcinomas that have not been found to yield sufficient results with BNCT using conventional BPA or that have not been considered to date a good indication for BNCT. In addition, the method and the use of the kit of the present invention do not require special instruments or procedures, and can be easily used in general hospitals and laboratories. Furthermore, according to the present invention, there is provided a method and a use of a kit for selecting an optimal boron formulation for each carcinoma/case. That is, by examining the expression pattern of the initial target proteins of the various boron formulations, the optimal boron formulation combination and dosage can be determined for each carcinoma/case, and the BNCT response rate can be improved. Thus, according to the present invention, the potential of BNCT can be expanded, failures in finding cases can be reduced, and personalized medicine of BNCT can be achieved.

### Brief Description of Drawings

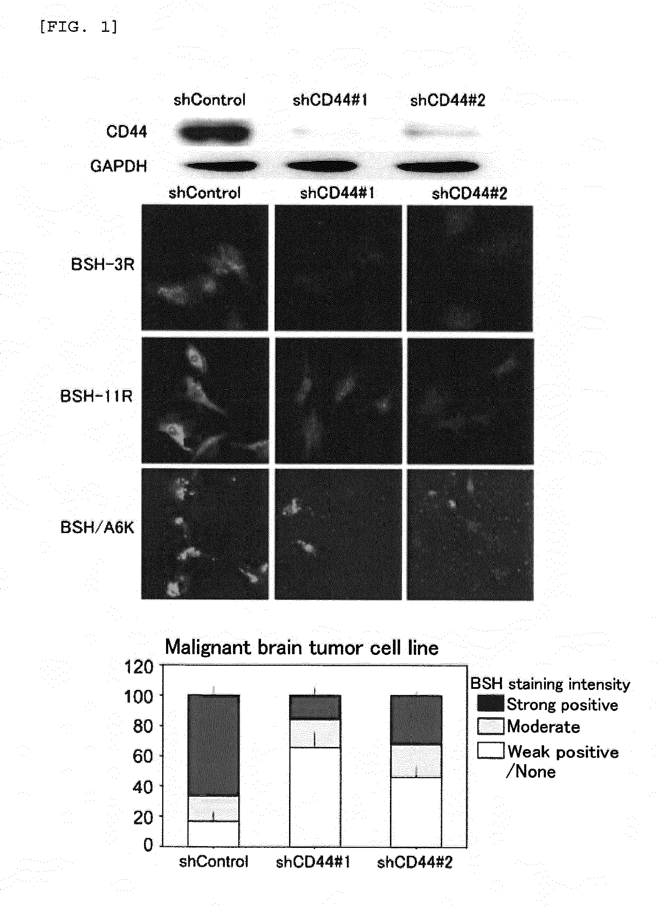

[Figure 1] Figure 1 is a diagram showing uptakes of BSH-3R, BSH-11R, and BSH/A6K complex into CD44-expressing cancer cells and CD44-knocked down cancer cells. shControl represents a malignant brain tumor cell line in which the CD44 gene is not knocked down (control), and shCD44 #1 and shCD44 #2 represent malignant brain tumor cell sublines in which the CD44 gene is knocked down. The upper section is the results of confirming the knockdown of CD44. The middle section is fluorescent immunostaining images of the control and the CD44-knocked down cell sublines with anti-BSH antibodies. The lower section is the breakdown (relative number) of anti-BSH antibody staining intensities of the control and the CD44-knocked down cell sublines when BSH-11R was used.

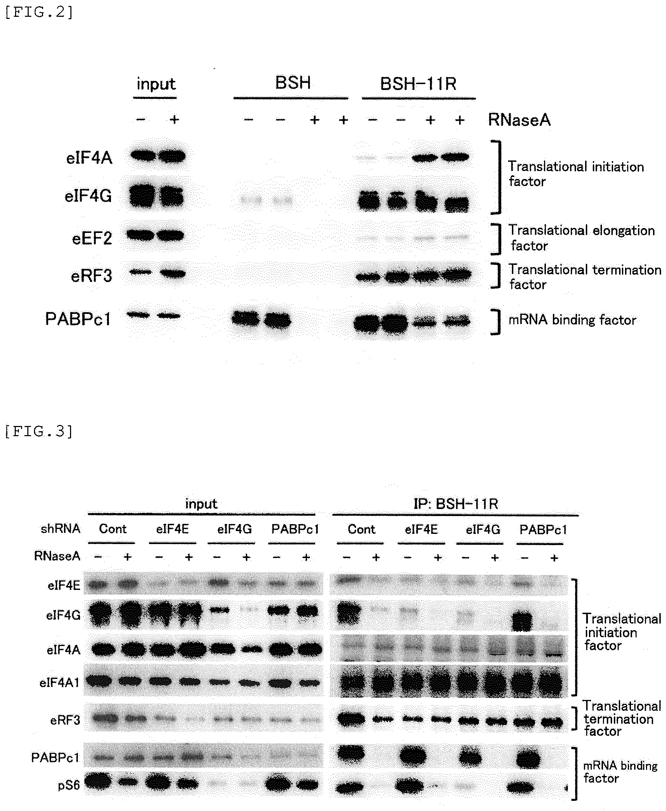

[Figure 2] Figure 2 is an immunoprecipitation pattern showing that BSH-11R targets a translation system in cancer cells.
[Figure 3] Figure 3 is an immunoprecipitation pattern showing that BSH-11R targets a translation system in cancer cells.

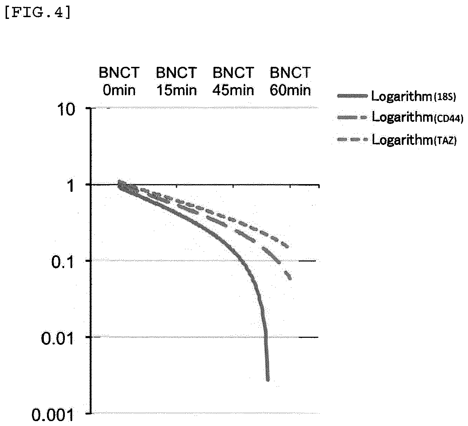

[Figure 4] Figure 4 is a graph showing the destruction of the translation system when cancer cells were pretreated with BSH-11R and subjected to BNCT. The solid line indicates logarithms of relative amounts of 18S ribosomal RNA, the broken line indicates logarithms of relative amounts of CD44 mRNA, and the dotted line indicates logarithms of relative amounts of TAZ mRNA.

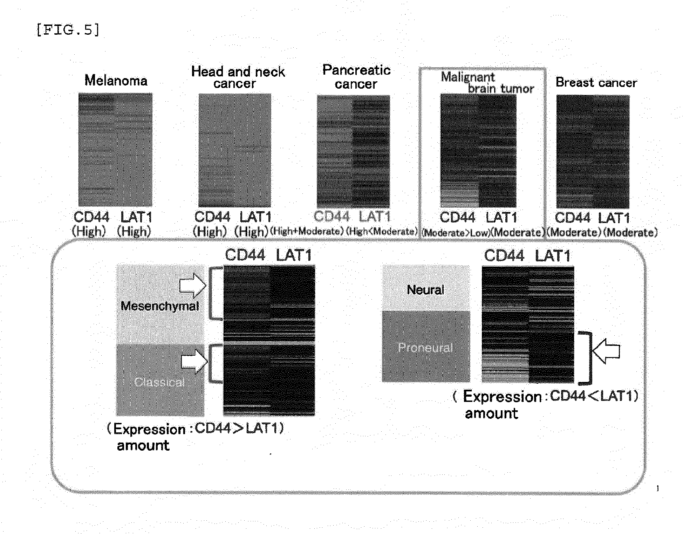

[Figure 5] Figure 5 shows the results of examining LAT1 and CD44 expressions in various carcinoma specimens (the upper section in the figure). Expressions of LAT1 and CD44 were examined for each type of malignant brain tumors (the lower section in the figure). In Figure 5, the higher the expression intensity, the more intense the red color, and the lower the expression intensity, the more intense the green color. When the expression intensity is moderate, it is indicated in black color. In the figure, one line shows the expression in one specimen.

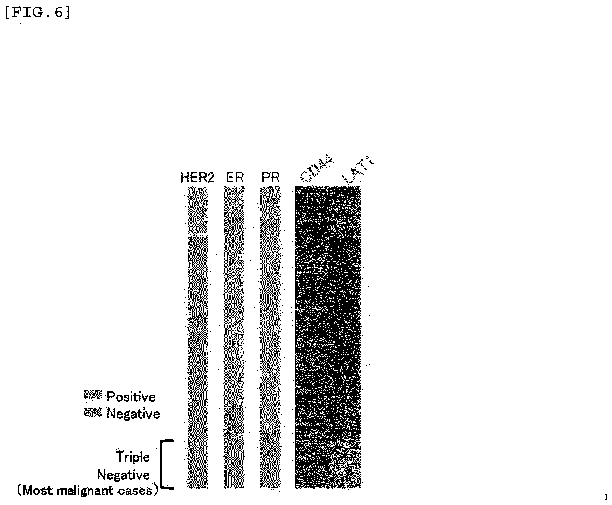

[Figure 6] Figure 6 shows the results of examining expressions of LAT1 and CD44 for each type of breast cancer. In Figure 6, the higher the expression intensity, the more intense the red color, and the lower the expression intensity, the more intense the green color. When the expression intensity is moderate, it is indicated in black color. In the figure, one line shows the expression in one specimen.

### Description of Embodiments

In the first aspect, the present invention provides a method for predicting efficiency of introduction into a cancer cell of a complex comprising a peptide containing a basic amino acid residue and BSH, or BSH to which a peptide containing a basic amino acid residue is covalently linked, the method comprising examining expression of CD44 in the cancer cell in a sample, wherein the peptide containing a basic amino acid residue contains an arginine residue and/or a lysine residue and is 2 to 20 amino acids in length.

CD44 is a cell membrane protein involved in cell adhesion, cell movement, and proliferation, infiltration, and metastasis of cancer cells, and the like. The present inventors have found for the first time that CD44 is a target for introduction into cells of a complex comprising a peptide containing a basic amino acid residue and BSH, and BSH to which a peptide containing a basic amino acid residue is covalently linked. That is, it has been found that a complex comprising a peptide containing a basic amino acid residue and BSH, and BSH to which a peptide containing a basic amino acid residue is covalently linked are easily introduced into cancer cells with high expression of CD44. Thus, in the above method, it can be predicted that the higher the expression of CD44 in a cancer cell, the higher the efficiency of introduction into a cancer cell of a complex comprising a peptide containing a basic amino acid residue and BSH, or BSH to which a peptide containing a basic amino acid residue is covalently linked.

The sample may be any sample as long as it contains a cancer cell. For example, the sample may be a pathological specimen or a biopsy specimen. Alternatively, the sample may be a body fluid sample, such as blood.

The cancer may be any type of cancer. Examples include, but are not limited to, epithelial cancers including a breast cancer, a pancreatic cancer, and an oral cancer, a brain tumor, and refractory cancers such as a bone and soft tissue tumor.

In the method of the present invention, the expression amount of CD44 in a cancer cell is examined. As used herein, CD44 means either one or both of a CD44 gene and a CD44 protein. Expression of CD44 can be examined by known methods. For example, expression of CD44 can be examined by detecting a CD44 protein in a cancer cell by immunostaining with an anti-CD44 antibody, or by examining expression of a CD44 gene in a cancer cell by Northern blot analysis or real-time PCR. Means and methods for examining expression of CD44 in a cancer cell are not limited thereto.

Examples of preferred methods for examining expression of CD44 include immunostaining with an anti-CD44 antibody. Anti-CD44 antibodies can be obtained by known methods and are also commercially available. The anti-CD44 antibody may be a polyclonal antibody or a monoclonal antibody, and preferably, a monoclonal antibody. Immunostaining is a known method. It is preferable to use an anti-CD44 antibody to which a detectable label such as a fluorescent dye, a fluorescent protein, or a radioisotope is attached, for immunostaining. Secondary antibodies may also be used to detect the anti-CD44 antibody. The anti-CD44 antibody may be directed to a CD44 protein or a portion thereof, or may be directed to a gene encoding a CD44 protein or a portion thereof.

Northern blot analysis or real-time PCR is also preferably used to examine expression of CD44. These methods are known to those skilled in the art.

Determination of whether CD44 expression is high can also be performed by known methods. For example, whether the expression of CD44 is high can be determined using the expression of CD44 in a cell from a normal subject or in a cell from a patient with a benign disease such as a benign tumor as a basis. Whether the expression of CD44 is high may also be determined using the expression amount of a housekeeping gene such as glyceraldehyde-3-phosphate dehydrogenase (GAPDH) or β-actin or of a product thereof as a basis.

A complex comprising a peptide containing a basic amino acid residue and BSH can be obtained by mixing, and conjugating a peptide containing a basic amino acid residue to BSH in an aqueous solution. Optionally, the size of the complex may be adjusted using, for example, an extruder having an appropriate pore size. The method for producing the complex comprising a peptide containing a basic amino acid residue and BSH may be a similar method to that described in International Patent Application Publication WO 2018/097335 A1.

BSH to which a peptide containing a basic amino acid residue is covalently linked can be obtained by known methods. For example, a peptide containing a basic amino acid residue may be covalently linked to BSH via an SH group of BSH.

The peptide containing a basic amino acid residue contains an arginine residue and/or a lysine residue and is 2 to 20 amino acids in length, preferably 3 to 16 amino acids, more preferably 4 to 14 amino acids, still more preferably 6 to 13 amino acids.

The peptide containing a basic amino acid residue, which forms a complex with BSH, is preferably a peptide containing a basic amino acid residue as described above and a hydrophobic amino acid residue. The hydrophobic amino acid residue is also known, and examples thereof include, but are not limited to, a glycine residue, an alanine residue, a valine residue, a leucine residue, an isoleucine residue, a methionine residue, a proline residue, a phenylalanine residue, a tryptophan residue, a tyrosine residue, and a cysteine residue.

As described above, the peptide containing a basic amino acid residue, which forms a complex with BSH, is a peptide containing an arginine residue and/or a lysine residue. Examples of the peptide containing a basic amino acid residue, which forms a complex with BSH, include, but are not limited to, AAAAAK, AAAAAAK, AAAAAAAK, AAAAAKK, AAAAAAKK, AAAAAAAKK, AAAAAR, AAAAAAR, AAAAAAAR, AAAAARR, AAAAAARR, AAAAAAARR, in one letter amino acid notation. More preferred examples of the peptide containing a basic amino acid residue, which forms a complex with BSH include, but are not limited to, AAAAAAK and AAAAAAR.

The peptide containing a basic amino acid residue, which is covalently linked to BSH, is a peptide containing an arginine residue and/or a lysine residue. It is preferred that the isoelectric point of the peptide containing a basic amino acid residue, which is covalently linked to BSH, is higher than 7. Alternatively, 50% or more, more preferably 60% or more, still preferably 70% or more, and still more preferably 80% or more of the amino acid residues constituting the peptide containing a basic amino acid residue, which is covalently linked to BSH, are basic amino acid residues. Preferred examples of the peptide containing such basic amino acid residues include, but are not limited to, 3 to 16 mers of arginine and 3 to 16 mers of lysine. More preferred examples of the peptide containing a basic amino acid residue, which is covalently linked to BSH, include, but are not limited to, 4 to 14 mers of arginine or 4 to 14 mers of lysine, still preferably 6 to 13 mers of arginine or 6 to 13 mers of lysine.

In the second aspect, the present invention provides the use of a kit for predicting efficiency of introduction into a cancer cell of a complex comprising a peptide containing a basic amino acid residue and BSH, or BSH to which a peptide containing a basic amino acid residue is covalently linked, the kit comprising means for examining expression of CD44. The kit can be used to perform the method of the first aspect. The means for examining expression of CD44 is known to those skilled in the art, and is not particularly limited, but preferred examples of the means include immunostaining with an anti-CD44 antibody, and expression analysis of a CD44 gene using Northern blot analysis or real-time PCR. The kit may thus comprise a reagent or an instrument for performing immunostaining with an anti-CD44 antibody. Alternatively, the kit may comprise a reagent or an instrument for Northern blot analysis or real-time PCR for examining expression of CD44. The kit typically comprises an instruction.

In the third aspect, the present invention provides a method for predicting a sensitivity of a cancer cell to a boron neutron capture therapy (BNCT) using a complex comprising a peptide containing a basic amino acid residue and BSH, or BSH to which a peptide containing a basic amino acid residue is covalently linked, the method comprising examining expression of CD44 in the cancer cell in a sample, wherein the peptide containing a basic amino acid residue contains an arginine residue and/or a lysine residue and is 2 to 20 amino acids in length.

Since the complex comprising a peptide containing a basic amino acid residue and BSH, and BSH to which a peptide containing a basic amino acid residue is covalently linked are easily introduced into cancer cells with high expression of CD44, cancer cells with high expression of CD44 are highly sensitive to BNCT using the complex comprising a peptide containing a basic amino acid residue and BSH and/or BSH to which a peptide containing a basic amino acid residue is covalently linked. Thus, in the above method, it can be predicted that the higher the expression of CD44 in a cancer cell, the higher the sensitivity of the cancer cell to BNCT using a complex comprising a peptide containing a basic amino acid residue and BSH, or BSH to which a peptide containing a basic amino acid residue is covalently linked.

In the fourth aspect, the present invention provides the use of a kit for determining a sensitivity of a cancer cell to BNCT using a complex comprising a peptide containing a basic amino acid residue and BSH, or BSH to which a peptide containing a basic amino acid residue is covalently linked, the kit comprising means for examining expression of an anti-CD44 antibody. The kit can be used to perform the method of the third aspect. The kit typically comprises an instruction.

According to the method of the third aspect and the use of the kit of the fourth aspect of the present invention, it is not only possible to know whether a cancer of a patient is a case suitable for BNCT using a complex comprising a peptide containing a basic amino acid residue and BSH and/or BSH to which a peptide containing a basic amino acid residue is covalently linked, but also to optimize the type of appropriate boron formulation in BNCT (for example, whether a complex comprising a peptide containing a basic amino acid residue and BSH and/or BSH to which a peptide containing a basic amino acid residue is covalently linked is effective, BPA is effective, or a combination thereof is effective) and to optimize the dosage or the compounding amount of the boron formulation.

For other details of the method of the third aspect and the use of the kit of the fourth aspect of the present invention, the descriptions for the method of the first aspect and the use of the kit of the second aspect of the present invention are applied.

As mentioned above, the higher the expression of CD44, the higher the efficiency of introduction of a complex comprising a peptide containing a basic amino acid residue and BSH, or BSH to which a peptide containing a basic amino acid residue is covalently linked. Then, the higher the expression of CD44, the higher the sensitivity to BNCT using a complex comprising a peptide containing a basic amino acid residue and BSH, or BSH to which a peptide containing a basic amino acid residue is covalently linked. Thus, with the examination of the expression of CD44 in a cancer cell, it can be predicted that the higher the expression of CD44, the higher the possibility that BNCT is effective using a complex comprising a peptide containing a basic amino acid residue and BSH, or BSH to which a peptide containing a basic amino acid residue is covalently linked. Accordingly, in the fifth aspect, the present invention provides a method for predicting a possibility that BNCT is effective using a complex comprising a peptide containing a basic amino acid residue and BSH, or BSH to which a peptide containing a basic amino acid residue is covalently linked, the method comprising: examining an expression of CD44 in a cancer cell in a sample; and predicting that the higher the expression of CD44, the higher the possibility that BNCT is effective using a complex comprising a peptide containing a basic amino acid residue and BSH, or BSH to which a peptide containing a basic amino acid residue is covalently linked.

Furthermore, in the sixth aspect, the present invention provides the use of a kit for predicting a possibility that BNCT is effective using a complex comprising a peptide containing a basic amino acid residue and BSH, or BSH to which a peptide containing a basic amino acid residue is covalently linked, the kit comprising means for examining expression of CD44. The kit can be used to perform the method of the ninth aspect. The kit typically comprises an instruction.

For the methods and the use of the kits according to the fifth and sixth aspect of the present invention, the descriptions for the methods and the use of the kits according to the first to fourth aspects are applied.

Since BPA is taken up into cells through amino acid transporters such as LAT1, LAT2, ATB^{0,+}, the uptake of BPA is higher as the expression of these amino acid transporters in a cancer cell is higher. Thus, for BPA in cancer cells, with the examination of the expression of an amino acid transporter such as LAT1, LAT2, or ATB^{0,+}, it can be predicted that the higher the expression of the amino acid transporter, the higher the possibility that BNCT is effective using BPA.

To summarize the above, the following can be said: the higher the expression of CD44 in a cancer cell, the more appropriate the selection of a boron formulation containing a complex comprising a peptide containing a basic amino acid residue and BSH, or BSH to which a peptide containing a basic amino acid residue is covalently linked. It can also be said that the higher the expression of an amino acid transporter such as LAT1, LAT2, ATB^{0,+} (preferably LAT1) in a cancer cell, the more appropriate the selection of a boron formulation containing BPA. Accordingly, in the seventh aspect, the present invention provides a method for selecting a boron formulation for a boron neutron capture therapy (BNCT), comprising the steps of:
(a) examining expression of CD44 and expression of LAT1 in a cancer cell in a sample; and then
(b) selecting a boron formulation containing a complex comprising a peptide containing a basic amino acid residue and BSH, or BSH to which a peptide containing a basic amino acid residue is covalently linked when the expression of CD44 is higher than the expression of LAT1, and selecting a boron formulation containing BPA when the expression of LAT1 is higher than the expression of CD44.

Furthermore, in the eighth aspect, the present invention provides the use of a kit for use in selecting a boron formulation for BNCT, the kit comprising means for examining expression of CD44 and means for examining expression of LAT1. The kit can be used to perform the method of the seventh aspect. The kit typically comprises an instruction.

In the methods and use of the kits of the present invention according to the seventh and eighth aspect, the expression amounts of CD44and LAT in a cancer cell are examined. The expression amounts of CD44 are examined as described above. As used herein, LAT1 means either one or both of a LAT1 gene and a LAT1 protein. Expression of LAT1 can be examined by known methods. For example, expression of LAT1 can be examined by detecting a LAT1 protein in a cancer cell by immunostaining with an anti-LAT1 antibody, or by examining expression of a LAT1 gene in a cancer cell by Northern blot analysis or real-time PCR. Means and methods for examining expression of LAT1 in a cancer cell are not limited thereto.

Examples of preferred methods for examining expression of LAT1 include immunostaining with an anti-LAT1 antibody. Anti-LAT1 antibodies can be obtained by known methods and are also commercially available. The anti-LAT1 antibody may be a polyclonal antibody or a monoclonal antibody, and preferably, a monoclonal antibody. Immunostaining is a known method. It is preferable to use an anti-LAT1 antibody to which a detectable label such as a fluorescent dye, a fluorescent protein, or a radioisotope is attached, for immunostaining. Secondary antibodies can also be used to detect the anti-LAT1 antibody. The anti-LAT1 antibody may be an antibody against a LAT1 protein or a portion thereof, or may be an antibody against a gene encoding a LAT1 protein or a portion thereof.

Determination of whether LAT1 expression is high can also be performed by known methods. For example, whether the expression of LAT1 is high may be determined using the expression of LAT1 in a cell from a normal subject, or in a cell from a patient with a benign disease such as a benign tumor as a basis. Alternatively, whether the expression of LAT1 is high may be determined using the expression amount of a housekeeping gene such as glyceraldehyde-3-phosphate dehydrogenase (GAPDH) or β-actin or of a product thereof as a basis.

In addition to or in place of expression of LAT1, expression of an amino acid transporter specific for tyrosine or phenylalanine, such as LAT2 and/or ATB^{0,+}, may be examined.

Means and methods for comparing expression of CD44 to expression of LAT1 are known to those skilled in the art. One example thereof is to use antibodies against these proteins, in which labels identifiable to each other are attached to the antibodies. For example, the antibody against CD44 may be labeled with a green fluorescent protein and the antibody against LAT1 may be labeled with a red fluorescent protein, and the expression of the two may be visually compared. Another example thereof is to compare the difference between expression of CD44 in a cancer cell and in a normal cell with the difference between expression of LAT1 in a cancer cell and in a normal cell, e.g., using a statistical method. A comparison of expression of a translation-related factor with expression of LAT1 can be performed in the same manner.

In the methods and use of the kits according to the seventh and eighth aspect, when the expression of CD44 is higher than the expression of LAT1, a boron formulation containing a complex comprising a peptide containing a basic amino acid residue and BSH, or BSH to which a peptide containing a basic amino acid residue is covalently linked is selected. When the expression of LAT1 is higher than the expression of CD44, a boron formulation containing BPA is selected. When BNCT is performed using the boron formulation selected by the methods and the use of the kits of these aspects, the selected formulation may be used alone, or may be used in combination or formulated with another boron formulation. For example, when BPA is selected, BPA may be used alone, or a combination of BPA and a complex comprising BSH and BPA may be used. The dosage of boron formulation, the ratio thereof to another boron formulation, and the like can be readily determined by a physician.

For other details of the methods and the use of the kits according to the seventh and eighth aspect of the present invention, the descriptions for the methods and the use of the kits according to the first to sixth aspects are applied.

The meaning of the terms used herein is the same as commonly understood in the art such as medicine, pharmacy, biology, unless otherwise specified.

Hereinafter, the present invention is described in more detail and specifically with Examples, but Examples should not be construed as any limitation of the scope of the present invention.

### Examples

### Example 1

Example 1. Uptake of BSH to which a peptide containing a basic amino acid residue is covalently linked and a complex comprising a peptide containing a basic amino acid residue and BSH, into a CD44-expressing cancer cell and a CD44-knocked down cancer cell

To examine the relationship between intracellular uptake of BSH to which a peptide consisting of three arginine is covalently linked (referred to as BSH-3R), BSH to which a peptide consisting of eleven arginine is covalently linked (referred to as BSH-11R), and a complex comprising Ala-Ala-Ala-Ala-Ala-Ala-Lys and BSH (referred to BSH/A6K) and CD44 expression, a CD44-shRNA lentivirus was first introduced into a malignant brain tumor cell line U87MG, which was highly expressing CD44, to produce a CD44-knocked down cell subline. The knockdown efficiency was confirmed by Western blotting method with an anti-CD44 antibody (manufactured by Cell Signaling Technology, Inc.). Then, to verify the correlation of the introduction efficiency of BSH-11R and BSH/A6K with the expression level of CD44, the intracellular uptake of BSH-11R and BSH/A6K into a CD44-knocked down cell subline produced by the method described above was examined by performing fluorescent immunostaining with an antibody against BSH. The antibody used was a mouse monoclonal anti-BSH antibody (provided by Dr. Kirihata, Osaka Prefecture University). BSH-3R and BSH-11R were obtained by chemical synthesis. BSH/A6K was obtained by the method described in International Patent Application Publication WO 2018/097335 A1.

The results are shown in Figure 1. As can be seen from western blotting shown in the upper section of Figure 1, CD44 was definitely knocked down in the produced two cell sublines. As shown in the middle section of Figure 1, it was confirmed that the lower the expression of CD44, the more reduction of the intracellular uptakes of BSH-3R, BSH-11R, and BSH/A6K (the lower section of Figure 1 is a bar graph of the result when BSH-11R was used). In other words, it was confirmed that the higher the expression of CD44, the more increase the intracellular uptake of BSH-3R, BSH-11R, and BSH/A6K has.

### Example 2 (Reference Example)

Example 2. Targeting of translation-related factors by BSH to which a peptide containing a basic amino acid residue is covalently linked

To a cell crushing solution of a malignant brain tumor cell line U251, simple BSH (referred to BSH) (50 µM) or BSH-11R (50 µM) was added, and proteins which bind to BSH and BSH-11R were explored by immunoprecipitation with an anti-BSH antibody (10 µg).

The results are shown in Figure 2. Since more translation-related factors were co-precipitated with BSH-11R compared to BSH, it was found that the peptide portion (11R) of BSH-11R is necessary for binding to translation-related factors. It was also found that there are mRNA-independent and mRNA-dependent binding patterns according to the translation-related factors.

To examine whether the binding of BSH-11R to the translation-related factors is a direct or indirect binding, a subline of malignant brain tumor cell line U251 that underwent knockdown of the translation-related factors with shRNA lentivirus was prepared. To the cell crushing solutions, BSH-11R (50 µM) was added, and translation-related factors that bind to BSH-11R were analyzed with an anti-BSH antibody (10 µg).

The results are shown in Figure 3. It was found that BSH-11R binds directly (without RNA) to eIF4A and eRF3, and eIF4E and eIF4G bind indirectly to BSH-11R via eIF4A. It was also concluded, as combined with the results in Figure 2, that PABPc1 binds to BSH in an RNA-dependent manner.

### Example 3 (Reference Example)

Example 3. BNCT using BSH to which a peptide containing a basic amino acid residue is covalently linked

From the binding of BSH-11R to translation-related factors, it was thought that the translation mechanism could be selectively targeted. It was thus decided to perform BNCT using BSH-11R. Malignant brain tumor cell lines U87ΔEGFR were pretreated with 100 µM BSH-11R (24 hours prior to irradiation). The cells were stripped with trypsin on the day of irradiation, dispensed into tubes at an amount of 1 x 10⁶ cells/mL, and irradiated with neutron rays using an accelerator (at the National Institute of Radiological Sciences). The irradiation times were 0, 15, 45 and 60 minutes, and every substantial Gy (Gray) was estimated and calculated to be less than or equal to 1 Gy.

The cells after irradiation were recovered, RNA extraction was performed, and then cDNA was produced using an equal amount of RNA (1000 ng) as a template, and quantitative PCR was performed. As the result, it was found as shown in Figure 4 that the proportion of 18S ribosomal RNA and mRNA decreased significantly depending on the irradiation time with neutron rays. No such changes were observed in high-dose irradiation (10 Gy) with X-rays performed as a control experiment. From these, it could be determined that BNCT using BSH-11R was able to destroy the translation mechanism extremely efficiently.

### Example 4

Example 4. Comparison of expression levels of LAT1 and CD44 in various carcinomas

Figure 5 shows the results of examining the expression intensities of CD44 and LAT1 for specimens of melanoma, head and neck cancer, pancreatic cancer, malignant brain tumor, and breast cancer (clinical specimens registered in the TCGA database) by an immunostaining method.

In melanoma and head and neck cancer, there were many cases in which both CD44 and LAT1 were highly expressed. From these results, it was considered that BNCT for melanoma and head and neck cancers responds in many cases when any one of a complex comprising a peptide containing a basic amino acid residue and BSH, BSH to which a peptide containing a basic amino acid residue is covalently linked, or BPA is selected. It was also considered that BNCT for melanoma and head and neck cancers is effective in many cases when a combination of a complex comprising a peptide containing a basic amino acid residue and BSH or BSH to which a peptide containing a basic amino acid residue is covalently linked with BPA is selected.

In pancreatic cancer, there were many cases in which CD44 was highly or moderately expressed. For LAT1, there were more cases with moderate expression than those with high expression. From these results, it was considered that BNCT for pancreatic cancer responds in many cases when a complex comprising a peptide containing a basic amino acid residue and BSH, or BSH to which a peptide containing a basic amino acid residue is covalently linked is selected, and that it responds to a certain degree in many cases when BPA is selected. It was also considered, in a combination use of a complex comprising a peptide containing a basic amino acid residue and BSH or a peptide containing a basic amino acid residue with BPA, that BNCT for pancreatic cancer is effective in many cases when the ratio of the complex comprising a peptide containing a basic amino acid residue and BSH or the peptide containing a basic amino acid residue is high.

In breast cancer, there were many cases in which both CD44 and LAT1 were moderately expressed. From these results, it was considered that BNCT for breast cancer is expected to respond to a certain degree in many cases when any one of a complex comprising a peptide containing a basic amino acid residue and BSH, BSH to which a peptide containing a basic amino acid residue is covalently linked, or BPA is selected. It was also considered that BNCT for breast cancer is expected to respond to a certain degree in many cases when a combination of a complex comprising a peptide containing a basic amino acid residue and BSH or BSH to which a peptide containing a basic amino acid residue is covalently linked with BPA is used.

In malignant brain tumors, for CD44, there were many cases with moderate expression, and also quite many cases with low expression (there were few cases with high expression). For LAT1, there were many cases with moderate expression. From these results, it was considered that BNCT for malignant brain tumors is expected to be effective to a certain degree in many cases when BPA is selected. It was also considered that BNCT for malignant brain tumors is expected to respond in fewer cases when a complex comprising a peptide containing a basic amino acid residue and BSH or BSH to which a peptide containing a basic amino acid residue is covalently linked is selected than when BPA is selected. Furthermore, it was considered, in a combination use of a complex comprising a peptide containing a basic amino acid residue and BSH or a peptide containing a basic amino acid residue with BPA, that BNCT for malignant brain tumors is effective in many cases when a ratio of BPA contained is high.

Malignant brain tumors were studied by type. The results are shown in the lower column of Figure 5. In mesenchymal and classical brain tumors, there are many cases in which CD44 is more highly expressed than LAT1 (indicated by arrows in the figure). It was thus considered that BNCT for them is effective in many cases when a complex comprising a peptide containing a basic amino acid residue and BSH, or BSH to which a peptide containing a basic amino acid residue is covalently linked is selected. It was also considered, in a combination use of a complex comprising a peptide containing a basic amino acid residue and BSH or a peptide containing a basic amino acid residue with BPA, BNCT for them is effective in many cases when the ratio of the complex comprising a peptide containing a basic amino acid residue and BSH or the peptide containing a basic amino acid residue is high. In neural brain tumors, there were many cases in which both CD44 and LAT1 were moderately expressed. In anterior neural brain tumors, there were many cases in which LAT1 is more highly expressed than CD44 (indicated by arrows in the figure), thus it was considered that BNCT for them is effective in many cases when BPA is selected. It was also considered, in a combination use of a complex comprising a peptide containing a basic amino acid residue and BSH or BSH to which a peptide containing a basic amino acid residue is covalently linked with BPA, that BNCT for them is effective in many cases when a ratio of BPA contained is high.

Breast cancer was also studied by type. The results are shown in Figure 6. In the most malignant cases where all of the HER2, estrogen and progesterone receptors are negative, there are many cases in which LAT1 is more highly expressed than CD44. It was thus considered that BNCT for them is effective in many cases when BPA is selected. It was also considered, in a combination use of a complex comprising a peptide containing a basic amino acid residue and BSH or BSH to which a peptide containing a basic amino acid residue is covalently linked with BPA, BNCT for them is effective in many cases when a ratio of BPA compounded is high.

General CD44 and LAT1 expression may be examined for each carcinoma, and expression of CD44 and LAT1 may be analyzed for each case. Personalized medicine of BNCT can be achieved by case-by-case analysis.

From the above results, it can be said that, with the methods and the use of the kits of the present invention, the optimal selection and determination of the combination or ratio of boron formulations can be performed for each carcinoma/case, and the potential of BNCT can be expanded for carcinoma/cases that have not previously been considered a good indication for BNCT using PBA.

This application claims the benefit of priority to Japanese Patent Application No. 2018-025216 filed on February 15, 2018.

### Industrial applicability

The present invention can be used in testing and research of cancer, research, development, and manufacture of anticancer agents, and the like.

## Claims

1. A method for predicting efficiency of introduction into a cancer cell of a complex comprising a peptide containing a basic amino acid residue and mercaptoundecahydrodecaborate (BSH), or BSH to which a peptide containing a basic amino acid residue is covalently linked, the method comprising examining expression of CD44 in the cancer cell in a sample, wherein the peptide containing a basic amino acid residue contains an arginine residue and/or a lysine residue and is 2 to 20 amino acids in length.

2. Use of a kit for predicting efficiency of introduction into a cancer cell of a complex comprising a peptide containing a basic amino acid residue and BSH, or BSH to which a peptide containing a basic amino acid residue is covalently linked, the kit comprising means for examining expression of CD44.

3. A method for predicting a sensitivity of a cancer cell to boron neutron capture therapy (BNCT) using a complex comprising a peptide containing a basic amino acid residue and BSH, or BSH to which a peptide containing a basic amino acid residue is covalently linked, the method comprising examining expression of CD44 in the cancer cell in a sample, wherein the peptide containing a basic amino acid residue contains an arginine residue and/or a lysine residue and is 2 to 20 amino acids in length.

4. Use of a kit for determining a sensitivity of a cancer cell to BNCT using a complex comprising a peptide containing a basic amino acid residue and BSH, or BSH to which a peptide containing a basic amino acid residue is covalently linked, the kit comprising means for examining expression of an anti-CD44 antibody.

5. A method for predicting a possibility that BNCT is effective using a complex comprising a peptide containing a basic amino acid residue and BSH, or BSH to which a peptide containing a basic amino acid residue is covalently linked, the method comprising: examining an expression of CD44 in a cancer cell in a sample; and predicting that the higher the expression of CD44, the higher the possibility that BNCT is effective using a complex comprising a peptide containing a basic amino acid residue and BSH, or BSH to which a peptide containing a basic amino acid residue is covalently linked.

6. Use of a kit for predicting a possibility that BNCT is effective using a complex comprising a peptide containing a basic amino acid residue and BSH, or BSH to which a peptide containing a basic amino acid residue is covalently linked, the kit comprising means for examining expression of CD44.

7. A method for selecting a boron formulation for a boron neutron capture therapy (BNCT), comprising the steps of:
(a) examining expression of CD44 and expression of LAT1 in a cancer cell in a sample; and then
(b) selecting a boron formulation containing a complex comprising a peptide containing a basic amino acid residue and BSH, or BSH to which a peptide containing a basic amino acid residue is covalently linked when the expression of CD44 is higher than the expression of LAT1, and selecting a boron formulation containing BPA when the expression of LAT1 is higher than the expression of CD44.

8. Use of a kit for selecting a boron formulation for BNCT according to the method of claim 7, the kit comprising means for examining expression of CD44 and means for examining expression of LAT1.

9. A complex comprising a peptide containing a basic amino acid residue and BSH, or BSH to which a peptide containing a basic amino acid residue is covalently linked for use in BNCT of a cancer, wherein the cancer highly expresses CD44.

10. The complex for use according to claim 9, wherein the peptide containing a basic amino acid residue contains an arginine residue and/or a lysine residue and is 2 to 20 amino acids in length.

## Patentansprüche

1. Verfahren zur Vorhersage der Wirksamkeit der Einführung eines Komplexes, der ein Peptid umfasst, das einen basischen Aminosäurerest und Mercaptoundecahydrodecaborat (BSH) enthält, oder BSH enthält, an das ein Peptid, das einen basischen Aminosäurerest enthält, kovalent gebunden ist, in eine Krebszelle, wobei das Verfahren die Untersuchung der Expression von CD44 in der Krebszelle in einer Probe umfasst, wobei das Peptid, das einen basischen Aminosäurerest enthält, einen Argininrest und/oder einen Lysinrest enthält und 2 bis 20 Aminosäuren lang ist.

2. Verwendung eines Kits zur Vorhersage der Wirksamkeit der Einführung eines Komplexes, der ein Peptid umfasst, das einen basischen Aminosäurerest und BSH enthält, oder BSH enthält, an das ein Peptid, das einen basischen Aminosäurerest enthält, kovalent gebunden ist, in eine Krebszelle, wobei der Kit Mittel zur Untersuchung der Expression von CD44 umfasst.

3. Verfahren zur Vorhersage der Sensitivität einer Krebszelle gegenüber der Bor-Neutroneneinfangtherapie (boron neutron capture therapy; BNCT) unter Verwendung eines Komplexes, der ein Peptid umfasst, das einen basischen Aminosäurerest und BSH enthält, oder BSH enthält, an das ein Peptid, das einen basischen Aminosäurerest enthält, kovalent gebunden ist, wobei das Verfahren die Untersuchung der Expression von CD44 in der Krebszelle in einer Probe umfasst, wobei das Peptid, das einen basischen Aminosäurerest enthält, einen Argininrest und/oder einen Lysinrest enthält und 2 bis 20 Aminosäuren lang ist.

4. Verwendung eines Kits zur Bestimmung der Sensitivität einer Krebszelle gegenüber BNCT unter Verwendung eines Komplexes, der ein Peptid umfasst, das einen basischen Aminosäurerest und BSH enthält, oder BSH enthält, an das ein Peptid, das einen basischen Aminosäurerest enthält, kovalent gebunden ist, wobei der Kit Mittel zur Untersuchung der Expression eines anti-CD44-Antikörpers umfasst.

5. Verfahren zur Vorhersage einer Möglichkeit, dass BNCT unter Verwendung eines Komplexes wirksam ist, der ein Peptid umfasst, das einen basischen Aminosäurerest und BSH enthält, oder BSH enthält, an das ein Peptid, das einen basischen Aminosäurerest enthält, kovalent gebunden ist, wobei das Verfahren umfasst: Untersuchung einer Expression von CD44 in einer Krebszelle in einer Probe; und Vorhersagen, dass umso höher die Expression von CD44, desto höher die Wahrscheinlichkeit ist, dass BNCT unter Verwendung eines Komplexes wirksam ist, der ein Peptid umfasst, das einen basischen Aminosäurerest und BSH enthält, oder BSH enthält, das mit einem Peptid, das einen basischen Aminosäurerest enthält, kovalent gebunden ist.

6. Verwendung eines Kits zur Vorhersage einer Möglichkeit, dass BNCT unter Verwendung eines Komplexes wirksam ist, der ein Peptid umfasst, das einen basischen Aminosäurerest und BSH enthält, oder BSH enthält, an das ein Peptid, das einen basischen Aminosäurerest enthält, kovalent gebunden ist, wobei der Kit Mittel zur Untersuchung der Expression von CD44 umfasst.

7. Verfahren zum Auswählen einer Bor-Formulierung für eine Bor-Neutroneneinfangtherapie (boron neutron capture therapy; BNCT), umfassend die Schritte:
(a) Untersuchen der Expression von CD44 und Expression von LAT1 in einer Krebszelle in einer Probe; und anschließend
(b) Auswählen einer Bor-Formulierung, die einen Komplex enthält, der ein Peptid umfasst, das einen basischen Aminosäurerest und BSH enthält, oder BSH enthält, an das ein Peptid, das einen basischen Aminosäurerest enthält, kovalent gebunden ist, wenn die Expression von CD44 höher als die Expression von LAT1 ist, und Auswählen einer Bor-Formulierung, die BPA enthält, wenn die Expression von LAT1 höher als die Expression von CD44 ist.

8. Verwendung eines Kits zum Auswählen einer Bor-Formulierung für BNCT gemäß des Verfahrens Anspruch 7, wobei der Kit Mittel zur Untersuchung der Expression von CD44 und Mittel zur Untersuchung der Expression von LAT1 umfasst.

9. Komplex, der ein Peptid umfasst, das einen basischen Aminosäurerest und BSH enthält, oder BSH enthält, an das ein Peptid, das einen basischen Aminosäurerest enthält, kovalent gebunden ist, zur Verwendung bei der BNCT einer Krebsart, wobei der Krebs CD44 hoch exprimiert.

10. Komplex zur Verwendung nach Anspruch 9, wobei das Peptid, das einen basischen Aminosäurerest enthält, einen Argininrest und/oder einen Lysinrest enthält und 2 bis 20 Aminosäuren lang ist.

## Revendications

1. Méthode pour prédire l'efficacité de l'introduction, dans une cellule cancéreuse, d'un complexe comprenant un peptide contenant un résidu d'acide aminé basique et du mercaptoundécahydrodécaborate (BSH), ou du BSH auquel un peptide contenant un résidu d'acide aminé basique est lié de manière covalente, la méthode comprenant l'examen de l'expression de CD44 dans la cellule cancéreuse dans un échantillon, dans laquelle le peptide contenant un résidu d'acide aminé basique contient un résidu arginine et/ou un résidu lysine et a une longueur de 2 à 20 acides aminés.

2. Utilisation d'un kit pour prédire l'efficacité de l'introduction, dans une cellule cancéreuse, d'un complexe comprenant un peptide contenant un résidu d'acide aminé basique et du BSH, ou du BSH auquel un peptide contenant un résidu d'acide aminé basique est lié de manière covalente, le kit comprenant des moyens pour examiner l'expression de CD44.

3. Méthode pour prédire la sensibilité d'une cellule cancéreuse à une thérapie par capture neutronique du bore (BNCT) utilisant un complexe comprenant un peptide contenant un résidu d'acide aminé basique et du BSH, ou du BSH auquel un peptide contenant un résidu d'acide aminé basique est lié de manière covalente, la méthode comprenant l'examen de l'expression de CD44 dans la cellule cancéreuse dans un échantillon, dans laquelle le peptide contenant un résidu d'acide aminé basique contient un résidu arginine et/ou un résidu lysine et a une longueur de 2 à 20 acides aminés.

4. Utilisation d'un kit pour déterminer la sensibilité d'une cellule cancéreuse à une BNCT utilisant un complexe comprenant un peptide contenant un résidu d'acide aminé basique et du BSH, ou du BSH auquel un peptide contenant un résidu d'acide aminé basique est lié de manière covalente, le kit comprenant des moyens pour examiner l'expression d'un anticorps anti-CD44.

5. Méthode pour prédire la possibilité qu'une BNCT utilisant un complexe comprenant un peptide contenant un résidu d'acide aminé basique et du BSH, ou du BSH auquel un peptide contenant un résidu d'acide aminé basique est lié de manière covalente, soit efficace, la méthode comprenant : l'examen de l'expression de CD44 dans une cellule cancéreuse dans un échantillon ; et la prédiction que plus l'expression de CD44 est élevée, plus la possibilité que la BNCT utilisant un complexe comprenant un peptide contenant un résidu d'acide aminé basique et du BSH, ou du BSH auquel un peptide contenant un résidu d'acide aminé basique est lié de manière covalente soit efficace est élevée.

6. Utilisation d'un kit pour prédire la possibilité qu'une BNCT utilisant un complexe comprenant un peptide contenant un résidu d'acide aminé basique et du BSH, ou du BSH auquel un peptide contenant un résidu d'acide aminé basique est lié de manière covalente, soit efficace, le kit comprenant des moyens pour examiner l'expression de CD44.

7. Méthode pour sélectionner une formulation de bore pour une thérapie par capture neutronique du bore (BNCT), comprenant les étapes de :
(a) examen de l'expression de CD44 et de l'expression de LAT1 dans une cellule cancéreuse dans un échantillon ; et ensuite
(b) sélection d'une formulation de bore contenant un complexe comprenant un peptide contenant un résidu d'acide aminé basique et du BSH, ou du BSH auquel un peptide contenant un résidu d'acide aminé basique est lié de manière covalente, quand l'expression de CD44 est plus élevée que l'expression de LAT1, et sélection d'une formulation de bore contenant du BPA quand l'expression de LAT1 est supérieure à l'expression de CD44.

8. Utilisation d'un kit pour sélectionner une formulation de bore pour une BNCT conformément à la méthode de la revendication 7, le kit comprenant des moyens pour examiner l'expression de CD44 et des moyens pour examiner l'expression de LAT1.

9. Complexe comprenant un peptide contenant un résidu d'acide aminé basique et du BSH, ou du BSH auquel un peptide contenant un résidu d'acide aminé basique est lié de manière covalente, pour une utilisation dans une BNCT d'un cancer, dans lequel le cancer exprime fortement CD44.

10. Complexe pour une utilisation selon la revendication 9, dans lequel le peptide contenant un résidu d'acide aminé basique contient un résidu arginine et/ou un résidu lysine et a une longueur de 2 à 20 acides aminés.
